# EUROPEAN PATENT SPECIFICATION

(11) **EP 2 870 977 B1**
(45) Date of publication and mention of the grant of the patent: **02.05.2018**
(21) Application number: 13813294.9
(22) Date of filing: 04.07.2013
(51) Int. Cl.: A61M 25/06, A61M 5/32, A61B 5/15, A61B 5/155, A61B 5/153

(54) **EXTRACTION DEVICE FOR COLLECTING BLOOD SAMPLES, INCLUDING A CATHETER AND A SAFETY SYSTEM**
EXTRAKTIONSVORRICHTUNG ZUM SAMMELN VON BLUTPROBEN MIT EINEM KATHETER UND EINEM SICHERHEITSSYSTEM
DISPOSITIF EXTRACTEUR/COLLECTEUR D'ÉCHANTILLONS DE SANG À CATHÉTER ET SYSTÈME DE SÉCURITÉ

(30) Priority: 04.07.2012 MX 2012007851
(43) Date of publication of application: 13.05.2015
(73) Proprietor: Equipos Medicos Vizcarra, S.A., Emiliano Zapata, Morelos 62765 (MX)
(72) Inventor: RODRIGUEZ LELIS, José María, Emiliano Zapata Morelos 62765 (MX); ARELLANO CABRERA, José Antonio, Emiliano Zapata Morelos 62765 (MX); LUCAS JIMENEZ, María Teresa, Emiliano Zapata Morelos 62765 (MX); CHALITA VIZCARRA, Alfredo, Emiliano Zapata Morelos 62765 (MX)
(74) Representative: Temiño Ceniceros, Ignacio
(86) International application number: PCT/MX2013/000079
(87) International publication number: WO 2014/007601

(56) References cited:
- EP-A1- 1 386 633
- WO-A1-00/06221
- WO-A1-00/20058
- WO-A1-01/78595
- WO-A1-99/23947
- US-A1- 2003 176 843

## Description

### FIELD OF THE INVENTION

The present invention is related to the hospital instruments manufacturing industry. More specifically it is related to the industry of manufacturing instruments for extracting and collecting blood. Still more specifically, it is related to the industry of manufacturing instruments for extracting blood safely, by means of a needle-catheter system which prevents the accidental puncturing or cutting of the vein by replacing the needle for a plastic catheter without sharp edges, which also prevents accidental punctures following its use.

### BACKGROUND OF THE INVENTION

Phlebotomy is the process of making an incision in a vein and is generally related to venipuncture and the extraction of blood. For decades phlebotomy has been and today it is one of the most common invasive techniques in health care [1]. There are two types of systems used for collecting blood: (a) open systems in which a blood sample, once it has been extracted for the patient's vein, must be transferred to another container for later analysis, these systems including hypodermic needles and syringes; (2) closed systems in which the blood that is collected is stored in the final container for later analysis and in which there is no need for transferring the blood to another device. Closed systems for the extraction of blood are preferred over the open systems due to their safety [2].

In 1943 [3] the American Red Cross asked a business which manufactured hospital material to develop a disposable, sterile apparatus for extracting blood. Once bottled, the material had to be kept sterile for use in battlefields. The result was the creation of a vacuum device, which allowed for extraction of blood directly from the vein, using a double pointed needle. One of the tips was directly connected to the collecting tube, which was in a vacuum, while the other tip of the needle was inserted into the vein. The blood was sucked into the vacuum tube, constituting a vacuum system for extracting blood. Since its beginnings, this device has been improved and perfected, transforming the system for blood extraction into a safe, practical procedure, which provides better quality for the diagnostic model. Today this kind of device for extracting and collecting blood samples is very common.

On the other hand, there is worldwide epidemiological evidence that shows that the main concern about accidents in the use of instruments with sharp points and their disposal as infectious waste in hospitals is the transmission of the AIDS virus and more commonly Hepatitis B and C viruses. This is due to the wounds caused by needles contaminated with human blood. Therefore there has been a growing need for producers of devices for extracting blood to try to find systems, which prevent accidental puncture wounds with needles to the medical staff and other workers who handle hospital waste.

As a result, new products are designed to incorporate special covers for needles or mechanisms for retracting the needle into a protective chamber. Such devices are described, for example, in U.S. patents No. 5.356.392, 6.004.278, 6.102.894, 6.186.960, 2003/208.161 A1, 2005/187.493 A1, 7.513.887 B2, 2010/241.029 A1, 2011/166.476 A1. All of the devices described in these patents involve the use of a hollow needle, which punctures the patient's vein in order to obtain a blood sample and remains in the vein for the duration of the procedure. This process does not prevent the patient from being wounded by the movement of the needle within the vein and the only safety provided is after extracting the needle from the patient for final disposal as hazardous wastes.

The continuous use of metal cannulas, which remain in the vein during blood extraction, causes irritation to the inside of the vein by the pointed end of the cannula. This can be dangerous since phlebitis may occur and in some cases, excessive damage to the vein nullifies the procedure. Today, blood extraction is performed manually, making it necessary for the person who performs the extraction to use very refined techniques and in most cases special training in the use of the devices used as well as broad experience in order to avoid harming and thus affecting the patient when blood extraction is carried out. However, even with experience and expertise, the patient is often affected by pain, tissue damage, multi-punctures and general discomfort.

One method developed to avoid the need for keeping a metallic cannula with sharp tips in the vein is the use of plastic catheters, which have disposable needles which run through the catheter to puncture the vein and allow the insertion of a catheter. Later, the needle may be removed, leaving the catheter in place to use as a connection for an intravenous bag, bottle or stopper for later use. With this method there is a flexible object with no sharp points inside the vein, thus preventing excessive irritation to the walls of the vein and eliminating the possibility of damaging the vein with a sharp object and the consequential ineffectiveness of the procedure. Moreover, psychologically the stress of the patient and the medical staff is diminished with the use of a non-cutting point, producing an improved procedure and greater comfort for the patient.

In patents such as US patent 2003/208.161A1 can be found a design for devices that ensure that the cannula loses its sharp edges after use. This consists of two concentric nested tubes, in which one has a sharp tip and the other a tip that is not sharp. Here the sharp tip is counterposed to the point which is not sharp, so that during the puncturing procedure the sharp tip cuts the skin and when it enters the vein the inner tube is deployed so that it blocks the sharp tip of the exterior tube exposing a tip which is not sharp. This mechanism affords the vein with a degree of safety of not being damaged by the tip of the needle, but there is still a rigid metallic element present which may tear the vein.

WO0178595A1 discloses needle-bearing medical devices provided for fluid infusion, fluid transfusion, and inserting a guide wire. After use, the needle is shielded to render the contaminated needle safe to prevent inadvertent needle sticks. The device includes a housing and a needle having a sharpened tip. A biasing element biases the needle toward a position in which the sharpened tip is shielded.

WO0020058A1 discloses a needle apparatus that has a thin walled catheter enclosing a tubular needle having a sharp point, the catheter and the needle being relatively moveable longitudinally a short distance, from a first position where the needle projects from the catheter and to a second position at which the sharp point is located within the catheter. The needle remains in the fluid pathway of the needle apparatus at all times. The needle apparatus is arranged to pierce tissue when the catheter and the needle are in the first position, and has a flange connected to the needle. However, this document does not disclose a device comprising a puncturing cannula and a collecting cannula, both cannulas generating a double system, and being independent from each other but linked by a safety latch configurated to slide.

US2003176843A1 discloses a retractable safety needle apparatus. The apparatus includes a needle holder with opposite proximal and distal ends. A needle assembly is mounted in the needle holder for movement from a first position where a distal cannula projects distally beyond the needle holder and a second position where the distal cannula is entirely within the needle holder. A spring is provided for propelling the needle assembly proximally and into the second position. The needle assembly includes a deflectable actuator for releasably holding the needle assembly in the first position and against the biasing forces of the spring. Portions of the needle holder adjacent the distal end are tapered to facilitate alignment of the needle assembly and to provide visual cues for proper orientation and gripping locations.

As may be inferred from the text, there are certain characteristics and qualities that a device for extracting and collecting blood must possess, among them: (a) reduce or eliminate the use of a metallic cannula with sharp edges and which remains in the vein for the duration of the procedure; (b) the use of a plastic cannula or catheter for the extraction of blood samples which provides the patient greater comfort with the assurance that the sharp element has been disabled; (c) possess a safety system for medical personnel that assures that following the procedure, no accidental puncture wounds occur and (d) be easily handled. These are the characteristics claimed in the present invention.

### OBJECTIVES OF THE INVENTION

The main objective of the invention is to achieve a device for the extraction of blood, which avoids prolonged permanence of a cannula with sharp edges inside the vein, replacing the latter with a flexible plastic catheter, which avoids damaging the patient's vein.

Another objective is to have a safety system, which avoids accidental puncture wounds to medical personnel and janitorial workers, with a hollow needle contaminated with blood by way of confining the used needle within a rigid casing.

Still another objective is to have a device for collecting blood samples with ergonomic geometry and which is easy for the personnel in charge of carrying out the procedure of extracting and collecting blood to use,

And all of those objective and advantages which will become apparent upon reading the attached drawings which for illustrative, with non-limiting purposes, make up an integral part of the present description.

### BRIEF DESCRIPTION OF THE INVENTION

The present invention refers to a device for extracting and collecting blood samples, specifically to a system for extracting blood samples which replaces the use of a hollow needle with a flexible plastic catheter for extracting blood from the vein of a patient and in which the hollow needle after making the hole and inserting the catheter is confined in a rigid casing so that the device cannot be reused and also thus preventing accidental puncture wounds to medical personnel or others with the hollow needle contaminate with blood, thereafter proceeding to safe elimination of the device.

This invention refers to a double system which allows, through one of its components, a system for extracting blood samples which uses a flexible catheter without a point which remains in a patient's vein, thus avoiding wounds to the vein and in which the hollow needle is only used for inserting the catheter into the patient's vein and will be immediately withdrawn, removing the sharp tip from the vein. In the second component, a safety mechanism which once the procedure of extracting blood is finished, allows for withdrawing the contaminated cannula into a protection chamber thus avoiding later contact with the user or other persons, preventing accidental puncture wounds and the transmission of diseases.

Moreover, with a system which utilizes a flexible catheter instead of a hollow needle for taking blood samples, the stress produced by a sharp metallic object within the body is reduced, thus preventing serious wounds which may invalidate the procedure. The procedure with the use of a flexible catheter gives medical personnel greater confidence when carrying out blood extraction and gives patients more comfort.

Furthermore, the characteristics of the retracting system of the device assure there is no contact with the cannula after placing the catheter, and allows for its being placed at a safe distance within the protection chamber of the safety system.

The mechanism consists of two spaces joined at the ends of a hollow housing and in which one of the spaces holds a flexible catheter and the other space, located at the opposite end of the space of the catheter, holds a rubber cap. A cannula or hollow puncturing needle passes through the flexible catheter, which is connected to a safety latch located within the main housing. In the latch, the puncturing cannula is connected to another cannula or hollow collecting needle which permits the flow of blood towards the container tubes. The safety latch is fixed, by means of a safety lock head to the housing in a front anchoring slot and on slide guides of the housing and under pressure by a compressed spring in the front part, in the position called the charge state. Once the puncturing is carried out and the catheter and cannula are inserted into the vein, the safety latch is disconnected manually from the front anchoring slot and, due to the decompression of the spring, it slides inside the housing until the head of the safety latch is fixed in the rear anchoring slot. This process causes the puncturing cannula to withdraw from the vein and remain within the main housing, and the collecting cannula is now located within the rubber cap. The configuration catheter-puncturing cannula-safety latch-collecting cannula creates a flow channel to lead blood to be deposited once the vacuum collecting tube is in place.

Once the process of collecting blood samples is completed, the catheter of the device is removed from the vein leaving only a flexible component, without a point, exposed, while the cannula or hollow needle is confined within the rigid housing, thus avoiding any accidental puncture wounds which could occur to anyone who has contact with the device during the disposal process.

### BRIEF DESCRIPTION OF THE DRAWINGS

Figure 1 shows an isometric view of the Extraction Device for Collecting Blood Samples including a Catheter and Safety System, from here on out called EDCBSCSS.
Figure 2 is an exploded drawing showing the parts that make up the EDCBSCSS.
Figure 3 shows the details of the protective tube and primary housing of the EDCBSCSS.
Figure 4 shows a top and side view of the EDCBSCSS and the way in which the front protective tube is connected.
Figure 5 shows an isometric image of the EDCBSCSS connected to the tube holder and the way the front protective tube is uncoupled.
Figure 6 shows an isometric image of the EDCBSCSS, with the cannula retracted, and the retracting system of the puncturing cannula is shown in detail.

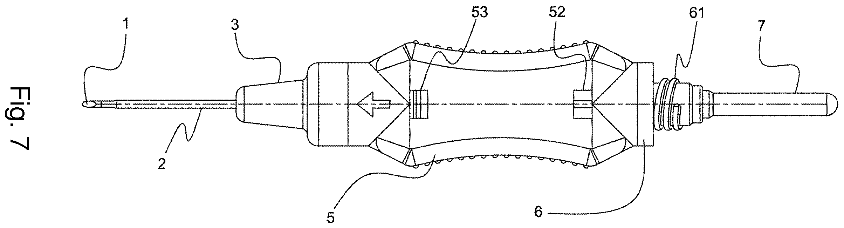

Figure 7 shows a lateral cut A-A in which details of EDCBSCSS in a charged state are shown.
Figure 8 shows a lateral cut A-A in which details of the EDCBSCSS in retraction and collecting of blood are shown.
Figure 9 shows an isometric image of the space of the cap.
Figure 10 shows an isometric image of the safety latch of the cannula holder.
Figure 11 shows an isometric image of the set of safety latches of the cannula in their final configuration.

### DETAILED DESCRIPTION OF THE INVENTION

For the detailed description we will refer to the figures of the preferred version of the present invention.

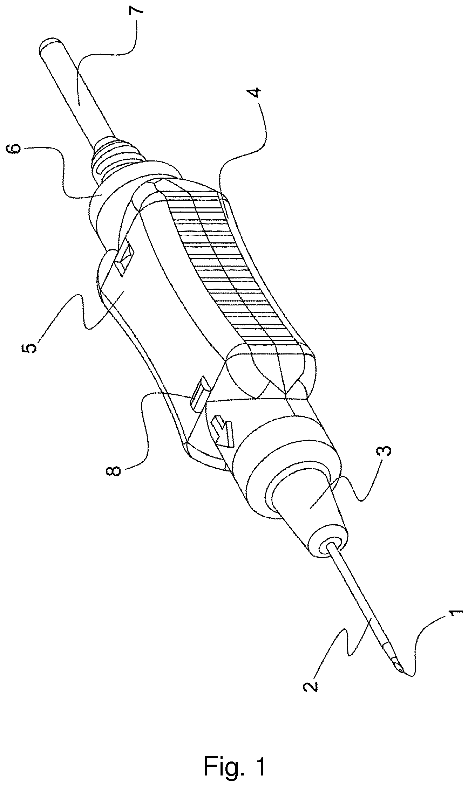

An isometric view of the Extraction device for collecting blood samples including a catheter and a safety system is shown in Figure 1. The catheter (2) surrounds the puncturing cannula (1). The catheter (2) is connected to the catheter space (3) and this in turn is connected to the housing components (5, 4). The puncturing cannula (1) is connected to the safety latch (8) which is within the housings components (5, 4). At the opposite end of the catheter space (3) the cap space (6) is connected. The cap space (6) is connected to the cap (7). A spring (9) can be found between the safety latch (8) and the catheter space (3) and the spring (9) pushes the safety latch (8) once it is free, in order to retrieve the puncturing cannula (1) and the safety latch (8) houses the collecting cannula (10) within the cap (7) and through the cap space (6). The group of catheter space (3), cap space (6) and housing components (5, 4) form a single unit when assembled.

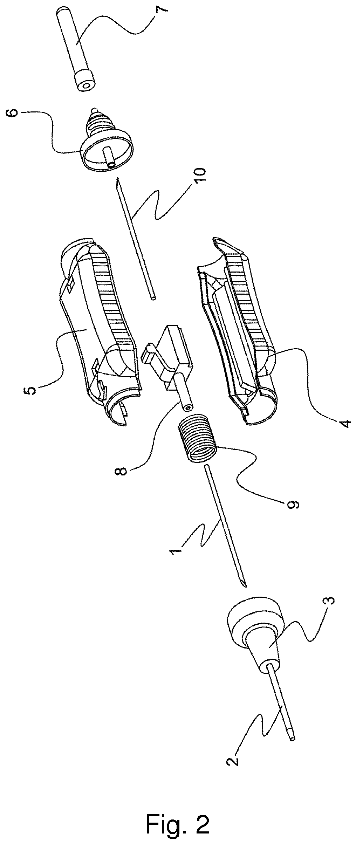

The EDCBSCSS consists of 10 elements as shown in the exploded view in Figure 2. As may be seen in this Figure 2, the parts which make up the EDCBSCSS are: puncturing cannula (1); catheter (2); catheter space (3); top housing (4); bottom housing (5); cap space (6); cap (7); cannula port safety latch (8); spring (9) and collecting cannula (10).

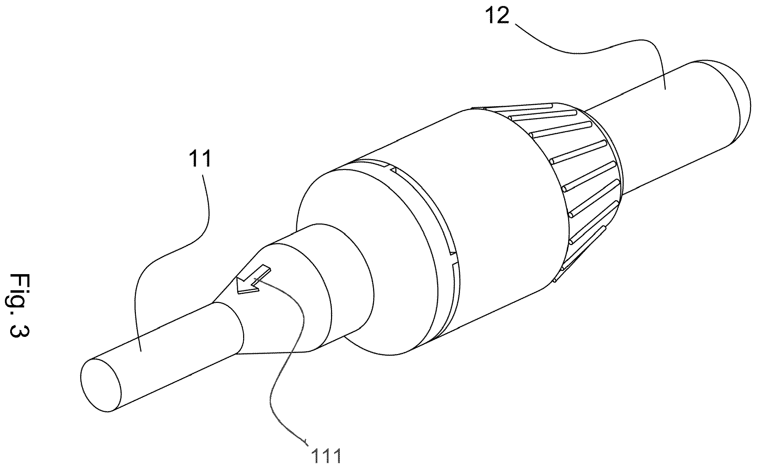

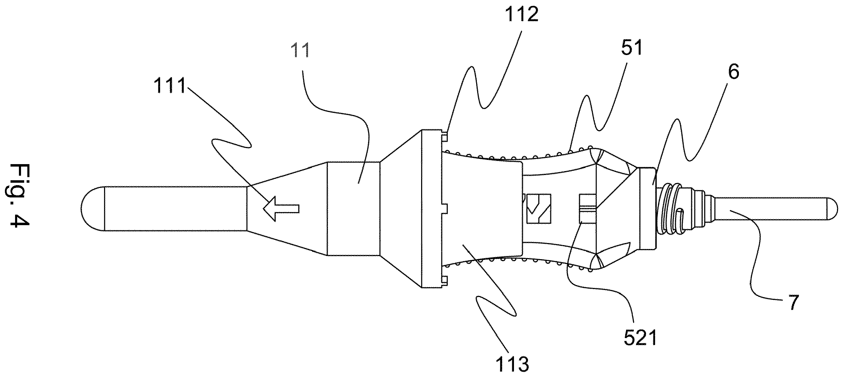

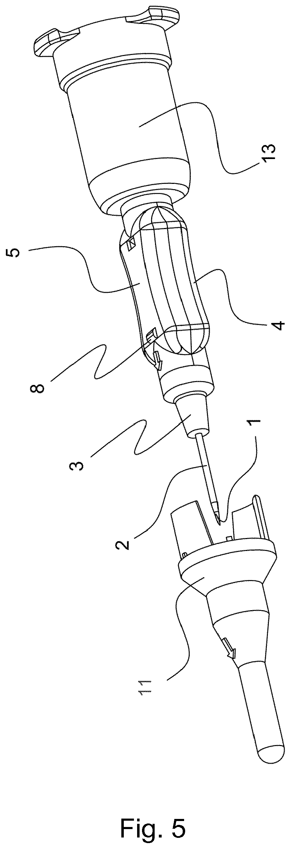

For transporting, the EDCBSCSS is housed in the protective tubes (11, 12) shown in Figure 3, whose function is to protect the EDCBSCSS during moving and prevent any contact at all with the needle before the EDCBSCSS is used. It is also equipped with the design characteristics necessary for sterilization. When the protective tube (12) is removed, part of the housings (4, 5), the cap space (6) and the cap (7) are exposed, and only the front protector (11) remains in place, covering the catheter (2), puncturing cannula (1) and the catheter space (3), as show in Figure 4. In this way the device is ready to be connected to the tube holder (13) and the EDCBSCSS is now ready for the puncturing and blood extraction procedure, as shown in Figure 5. In Figure 4 we can see details of the front protecting tube (11). These details ease the use of EDCBSCSS. The front protecting tube (11) present an indicator in the shape of an arrow (111) which lets the user know the direction and position of the tip of the puncturing cannula (1). Also, the front protecting tube has two wings (113) which connect to the rear protecting tube, leaving the handling curvatures (51) free, making it unnecessary to remove the front protecting tube until the procedure begins.

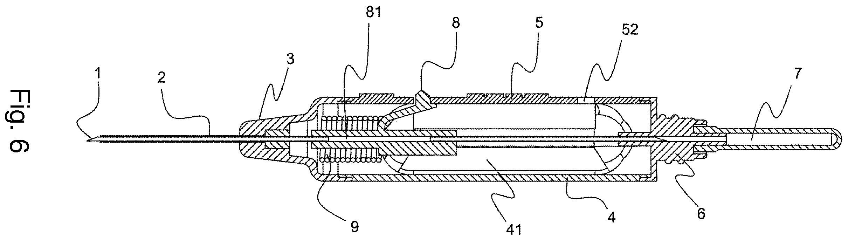

The detail of the EDCBSCSS in the charge state is shown in Figure 6. The puncturing cannula (1) and the collecting cannula (10) are linked to the safety latch (8) which is fixed inside the housing components (4, 5). The safety latch (8) is pushed by the spring (9) located between the safety latch (8) and the catheter space (3). In this charge state the puncturing cannula (1) is within the flexible catheter (2), while the collecting cannula (10) is outside the rubber cap (7) but inside the pivot guide (63) of the cap space (6). In this position the EDCBSCSS is ready for the puncturing procedure and the placing of the catheter (2) in order to proceed with extracting the blood. Once the catheter (2) is in the vein, the retraction mechanism of the puncturing cannula (1) is activated and the collection cannula (10) is ready for collecting the blood sample.

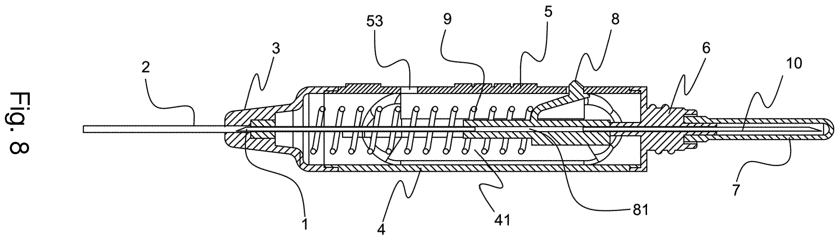

Figure 8 shows the details of the EDCBSCSS in the retraction and blood collecting state. Once the puncturing has taken place and the catheter (2) and puncturing cannula (1) are in the patient's vein, the retraction mechanism of the EDCBSCSS may be activated. This is done by pushing the top housing (5) towards the head of the safety latch (82) which due to its geometric conditions will flex the neck of the safety latch (85) and disconnect the housing (5). At the same time it will be shifted due to the decompression of the spring (9). The safety latch (8) will shift within the housings (4) and (5) guided by the housing guides (41) until it connects by way of the safety latch head (82) with the rear anchoring slot (52), remaining fixed in this position. The puncturing cannula (1), upon being attached to the safety latch (8) will move together with it, so that only the catheter (2) remains inside the vein while the collecting cannula (10) joined to the safety latch (8) will slide inside the cap (7), habilitating the system for collecting blood. In the configuration the extraction of blood of the EDCBSCSS forms a flow channel made up of the catheter (2), puncturing cannula (1), safety latch flow channel (81) and collecting cannula (10). This channel presents the optimal geometric conditions for easy and efficient extraction of blood once the vacuum tube is connected to the collecting cannula (10).

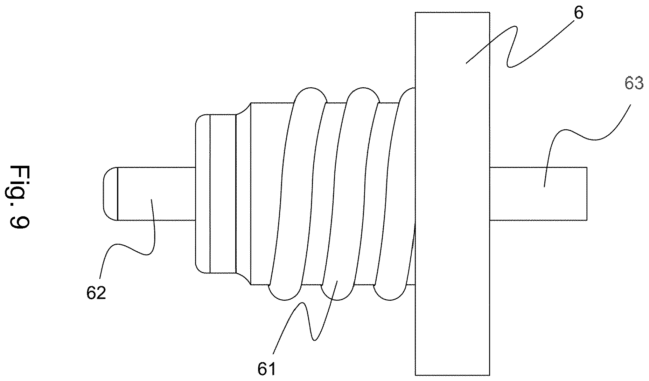

Figure 9 shows an isometric image of the cap space (6), which connects to the components, cap (7), the collecting cannula (10) and the tube holder (13) which makes up the collection system of the flow channel of the EDCBSCSS. The cap space (6) has standard threads (61) which are used to attach commercial tube holders for extracting blood samples, and also a hollow pivot (62) on the cap space (6) which is used to connect the cap (7), and on the opposite side there is a hollow pivot guide (63) for the collecting cannula (10) in which the collecting cannula (10) is stored and guided when the system for retracting the puncturing cannula (1) is retracted. The cap space (6) is joined to the housings (4, 5) in such a way as to form a single body.

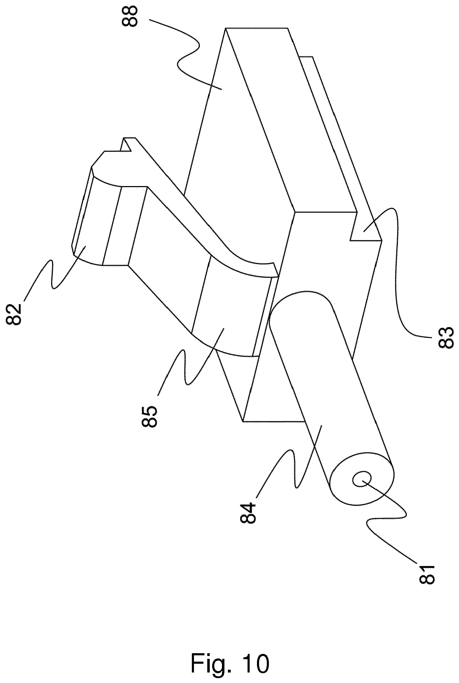

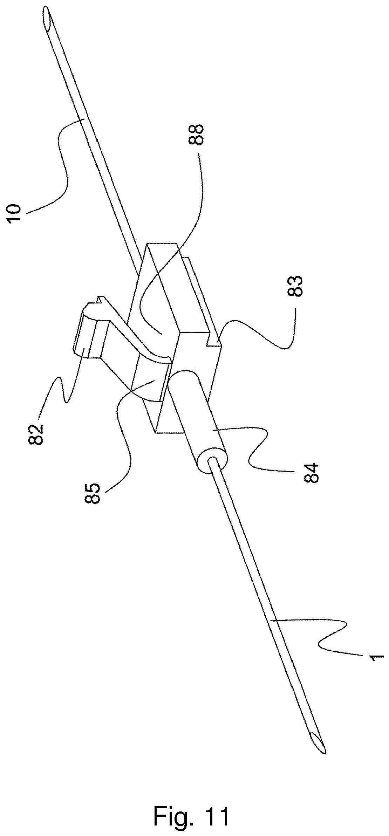

Figures 10 and 11 show details of the system for retracting the puncturing cannula (1) of the flow channel of the EDCBSCSS. The safety latch (8) consists of a rectangular base (88) with two slide guides (83) in the lower part located on each side of the rectangular base (88). These guides along with the housing guides (41) control the direction of the displacement of the safety latch (8). On the top part of the rectangular base (88) a pliable neck (85) protrudes on whose tip is the head (82) of the safety latch (8) whose function is to insert in the front anchoring slot (53) to conform the charge state of the EDCBSCSS. Once the reaction system is activated, the head (82) of the safety latch (8) is inserted into the rear anchoring slot (52) in order to activate the collection and safety system of the EDCBSCSS. On the front of the safety latch (8) a puncturing cannula pivot (84) protrudes and this supports and anchors the puncturing cannula (1) and acts as a guide for placing the spring (9). This pivot is hollow and constitutes a safety latch flow channel (81) which connects the puncturing cannula (1) to the collecting cannula (10) and together with the catheter (2) constitute the flow channel for extracting and collecting the blood sample towards the vacuum tube collector.

### References

[1] Lavery I, Ingram P. Blood sampling: best practice. Nursing Standard, 2005, 19:55 65.
[2] Berkeris L et al. Trends in blood culture contamination. A College of American Pathologist Q-tracks study of 356 institutions. Archives of Pathology and Laboratory Medicine, 2005, 123:1222-1226.
[3] McCALL, R. E.; TANKERSLEY, C. M. Phlebotomy Essentials. Philadelphia, Lippincott Williams & Wilkins, 3rd ed., 2003.

The invention has been described sufficiently so that a person with knowledge in the field may reproduce and obtain the results, which we mention in the present invention. However, anyone skilled in the art of the present invention may be capable of making modifications not described in the present application. If for the application of these modifications in a determined structure or in the process of manufacturing, the matter claimed in the following claims is required, said structures are considered within the scope of the invention.

## Claims

1. An extraction device for collecting blood samples, including a catheter and a safety system wherein the extraction device comprises a flexible catheter (2) which encloses a puncturing cannula (1); the catheter (2) surrounds the puncturing cannula (1) and is connected to a catheter space (3); wherein said catheter space (3) is connected to housing components (4, 5); said puncturing cannula (1) is connected to a safety latch (8) which is within the housing components (4, 5); wherein a spring (9) is located between the safety latch (8) and the catheter space (3), and it is configured to push the safety latch (8) once the safety latch (8) is free, in order to retrieve the puncturing cannula (1); wherein a cap space (6) is connected to an end of the housings (4, 5) opposite to the catheter space (3), and being said cap space (6) connected to a cap (7); and wherein the catheter space (3), the housing components (4, 5), the cap space (6) and the cap (7) are configured together to form a single body; and
**characterized in that** it comprises
a collecting cannula (10), for collecting a blood sample, housed by the safety latch (8) within the cap (7) and through an inside of a pivot guide (63) of the cap space (6); and wherein said collecting cannula (10) is linked to the puncturing cannula (1) by the safety latch (8) which is configurated to slide inside the housing components (4, 5).

2. The extraction device as defined in claim 1 wherein, the housing component (5), a head (82) of the safety latch (8); and a neck (85) of the safety latch (8) are configured so that, when activated, the safety latch (8) is disconnected from the housing component (5) and slides due to the decompression of the spring (9), the safety latch (8) shifting within the housing components (4, 5) guided by housing guides (41) until the safety latch (8) is connected by way of the safety latch head (82) with a rear anchoring slot (52), the safety latch (8) remaining fixed in this position; and wherein
the puncturing cannula (1), upon being attached to the safety latch (8) is moved together with the safety latch (8), so that only the catheter (2) remains inside a vein while the collecting cannula (10) joined to the safety latch (8) is slid inside the cap (7) which constitutes the system for blood collection.

3. The extraction device as defined in any of the claims 1 and 2, wherein, the safety latch (8) comprises a rectangular base (88) with two slide guides (83) in the lower part located on each side of the rectangular base (88), wherein the guides (83) along with housing guides (41) control a direction of displacement of the safety latch (8);
on a top part of the rectangular base (88), a pliable neck (85) protrudes and on a tip of the pliable neck (85) is a head (82) of the safety latch (8); wherein a function of the head of the safety latch (82) is to be inserted in a front anchoring slot (53) to conform the charge state of the extraction device, and also the head of the safety latch (82) is inserted into a rear anchoring slot (52) in order to activate the collection and safety systems of the extraction device.

4. The extraction device as defined in any of claims 1 to 3, **characterized by** having a safety system in which housing components (4, 5) act simultaneously with the safety latch (8), the puncturing cannula (1) and the spring (9).

## Patentansprüche

1. Extraktionsvorrichtung zum Sammeln von Blutproben mit einem Katheter und einem Sicherheitssystem, wobei die Extraktionsvorrichtung einen flexiblen Katheter (2) umfasst, welcher eine Punktionskanüle (1) umgibt; wobei der Katheter (2) die Punktionskanüle (1) umgibt und mit einem Katheterraum (3) verbunden ist; wobei der genannte Katheterraum (3) mit Gehäusebauteilen (4, 5) verbunden ist; die genannte Punktionskanüle (1) ist mit einer Sicherheitsklinke (8) verbunden, welche innerhalb der Gehäusebauteile (4, 5) liegt; wobei sich eine Feder (9) zwischen der Sicherheitsklinke (8) und dem Katheterraum (3) befindet, und dazu ausgebildet ist, die Sicherheitsklinke (8) zu drücken, sobald die Sicherheitsklinke (8) frei ist, um die Punktionskanüle (1) zurückzuziehen; wobei ein Kappenraum (6) mit einem Ende der Gehäuse (4, 5) dem Katheterraum (3) entgegengesetzt verbunden ist; und wobei der genannte Kappenraum (6) mit einer Kappe (7) verbunden ist; und wobei der Katheterraum (3), die Gehäusebauteile (4, 5), der Kappenraum (6) und die Kappe (7) dazu ausgebildet sind, zusammen einen einzelnen Körper zu bilden; und
**dadurch gekennzeichnet, dass** sie Folgendes umfasst:
eine Sammelkanüle (10), zum Sammeln von einer Blutprobe, welche mittels der Sicherheitsklinke (8) innerhalb der Kappe (7) und durch das Innere einer Schwenkführung (63) des Kappenraums (6) aufgenommen ist; und
wobei die genannte Sammelkanüle (10) mit der Punktionskanüle (1) mittels der Sicherheitsklinke (8) verknüpft ist, welche dazu ausgebildet ist, innerhalb der Gehäusebauteile (4, 5) zu gleiten.

2. Extraktionsvorrichtung nach Anspruch 1, wobei
der Gehäusebauteil (5), ein Kopf (82) der Sicherheitsklinke (8); und ein Hals (85) der Sicherheitsklinke (8) derart ausgebildet sind, dass, wenn sie aktiviert werden, die Sicherheitsklinke (8) vom Gehäusebauteil (5) gelöst wird und aufgrund der Entspannung der Feder (9) gleitet, wobei sich die Sicherheitsklinke (8) innerhalb der Gehäusebauteile (4, 5) verlagert, von Gehäuseführungen (41) geführt, bis die Sicherheitsklinke (8) mittels des Sicherheitsklinkenkopfs (82) mit einem hinteren Verankerungsschlitz (52) verbunden wird, wobei die Sicherheitsklinke (8) in dieser Stellung fixiert bleibt; und wobei
die Punktionskanüle (1), nachdem sie an der Sicherheitsklinke (8) befestigt wird, zusammen mit der Sicherheitsklinke (8) bewegt wird, so dass nur der Katheter (2) innerhalb einer Vene bleibt, während die mit der Sicherheitsklinke (8) zusammengefügten Sammelkanüle (10) innerhalb der Kappe (7) gleitet, welche das System zur Blutsammlung ausbildet.

3. Extraktionsvorrichtung nach einem der Ansprüche 1 und 2, wobei
die Sicherheitsklinke (8) eine rechteckige Basis (88) mit zwei Gleitführungen (83) im unteren Teil umfasst, welche sich an jeder Seite der rechteckigen Basis (88) befinden, wobei die Führungen (83) zusammen mit Gehäuseführungen (41) eine Verschiebungsrichtung der Sicherheitsklinke (8) steuern;
auf einem oberen Teil der rechteckigen Basis (88), ein biegsamer Hals (85) hervorsteht und auf einer Spitze des biegsamen Halses (85) ein Kopf (82) der Sicherheitsklinke (8) liegt; wobei eine Funktion des Kopfes der Sicherheitsklinke (82) ist, in einen vorderen Verankerungsschlitz (53) eingeführt zu werden, um den Ladungszustand der Extraktionsvorrichtung zu erfüllen, und auch der Kopf der Sicherheitsklinke (82) wird in einen hinteren Verankerungsschlitz (52) eingeführt, um die Sammlungs- und Sicherheitssysteme der Extraktionsvorrichtung zu aktivieren.

4. Extraktionsvorrichtung nach einem der Ansprüche 1 bis 3, **dadurch gekennzeichnet, dass** sie ein Sicherheitssystem aufweist, in welchem die Gehäusebauteile (4, 5) gleichzeitig mit der Sicherheitsklinke (8), der Punktionskanüle (1) und der Feder (9) wirken.

## Revendications

1. Dispositif d'extraction pour prélever des échantillons sanguins, comprenant un cathéter et un système de sécurité dans lequel le dispositif d'extraction comprend: un cathéter souple (2) qui contient une canule de perforation (1) ; le cathéter (2) entoure la canule de perforation (1) et est connecté à l'espace du cathéter (3) ; dans lequel ledit espace du cathéter est relié aux composants de logement (4, 5) ; ladite canule de perforation (1) est connectée au verrou de sécurité (8) qui se trouve à l'intérieur des composants de logements (4, 5) ; dans lequel un ressort (9) est situé entre le verrou de sécurité (8) et l'espace de cathéter (3), et il est configuré pour pousser le verrou de sécurité (8) une fois que le verrou de sécurité (8) est libre, afin de récupérer la canule de perforation (1) ;dans lequel un espace de bouchon (6) est connecté à une extrémité des logements (4, 5) opposée à l'espace de cathéter, et ledit espace de bouchon (6) étant connecté à un bouchon (7) ; et dans lequel l'espace de cathéter (3), les composants de logement (4, 5), l'espace de bouchon (5) et le bouchon (7) sont configurés ensemble pour former un simple corps ; et
**caractérisé en ce qu'**il comprend
une canule collectrice (10) pour prélever un échantillon sanguin, logée par le verrou de sécurité (8) dans le bouchon (7) et à travers l'intérieur d'un guide pivotant (63) de l'espace de bouchon (6) ; et
dans lequel ladite canule collectrice (10) est reliée à la canule de perforation (1) par le verrou de sécurité (8) qui est configuré pour glisser à l'intérieur des composants de logements (4, 5).

2. Dispositif d'extraction défini dans la revendication 1, dans lequel les composants de logement (5), une tête (82) du verrou de sécurité ; et un boulot (85) du verrou de sécurité (8) sont configurés de manière que, lorsqu'il est activé, le verrou de sécurité (8) est déconnecté du composant de logement (5) et glisse à cause de la décompression du ressort (9), le verrou de sécurité (8) se déplaçant dans les composants de logement (4, 5) guidé par les guides de logement (41) jusqu'à ce que le verrou de sécurité (8) est relié par le biais de la tête de verrou de sécurité (82) avec une rainure d'ancrage arrière (52), le verrou de sécurité (8) demeurant fixe dans cette position ; et dans lequel
la canule de perforation (1), lorsqu'elle est fixée au verrou de sécurité (8) elle se déplace conjointement avec le verrou de sécurité (8), de manière que seulement le cathéter (2) demeure à l'intérieur d'une veine tandis que la canule collectrice (10) reliée au verrou de sécurité (8) glisse à l'intérieur du bouchon (7) qui constitue le système de prélèvement sanguin.

3. Dispositif d'extraction défini dans l'une quelconque des revendications 1 et 2, dans lequel le verrou de sécurité (8) comprend une base rectangulaire (88) avec deux guides glissants (83) dans la partie inférieure située de chaque côté de la base rectangulaire (88), dans lequel les guides (83) conjointement avec les guides de logement (41) commandent la direction de déplacement du verrou de sécurité ;
sur une partie supérieure de la base rectangulaire (88), un cou pliant (85) fait saillie et sur une pointe du cou pliant (85) se trouve une tête (82) du verrou de sécurité (8) ; dans lequel une fonction de la tête du verrou de sécurité (82) est de s'insérer dans une rainure d'ancrage avant (53) pour conformer l'état de charge du dispositif d'extraction, et également la tête du verrou de sécurité (82) est insérée dans une rainure d'ancrage arrière (52) afin d'activer les systèmes de prélèvement et de sécurité du dispositif d'extraction.

4. Dispositif d'extraction défini dans l'une quelconque des revendications 1 à 3, **caractérisé en ce qu'**il comprend un système de sécurité dans lequel les composants de logements (4,5) agissent simultanément avec le verrou de sécurité (8), la canule de perforation (1) et le ressort (9).
